(19) Europäisches Patentamt
European Patent Office
Office européen des brevets

(11) Veröffentlichungsnummer: **0 279 357**
**A2**

(12) # EUROPÄISCHE PATENTANMELDUNG

(21) Anmeldenummer: 88102013.5

(51) Int. Cl.[4]: **A61K 37/64**, A61K 37/02

(22) Anmeldetag: 11.02.88

(30) Priorität: 19.02.87 DE 3705220

(43) Veröffentlichungstag der Anmeldung:
24.08.88 Patentblatt 88/34

(84) Benannte Vertragsstaaten:
AT BE CH DE ES FR GB GR IT LI LU NL SE

(71) Anmelder: BOEHRINGER INGELHEIM KG
Postfach 200
D-6507 Ingelheim am Rhein(DE)

(84) BE CH DE ES FR GR IT LI LU NL SE AT

(71) Anmelder: BOEHRINGER INGELHEIM INTERNATIONAL G.M.B.H.
Postfach 20
D-6507 Ingelheim am Rhein(DE)

(84) GB

(72) Erfinder: Schnorrenberg, Gerd, Dr.
Ernst-Ludwig-Strasse 66a
D-6535 Gau-Algesheim(DE)
Erfinder: Roos, Otto, Dr.
Elsheimer Strasse 36
D-6501 Schwabenheim(DE)
Erfinder: Lösel, Walter, Dr.
Im Herzenacker 26
D-6535 Gau-Algesheim(DE)
Erfinder: Arndts, Dietrich, Dr.
Mühlstrasse 7
D-6531 Appenheim(DE)
Erfinder: Speck, Georg, Dr.
Rheinstrasse 13
D-6507 Ingelheim/Rhein(DE)
Erfinder: Streller, Ilse, Dr.
Waldstrasse 16
D-6534 Stromberg(DE)

(54) **Mittel zur Behandlung von Coronarerkrankungen oder organischem Hirnsyndrom.**

(57) Die Verwendung einer Verbindung gemäß Formel I

EP 0 279 357 A2

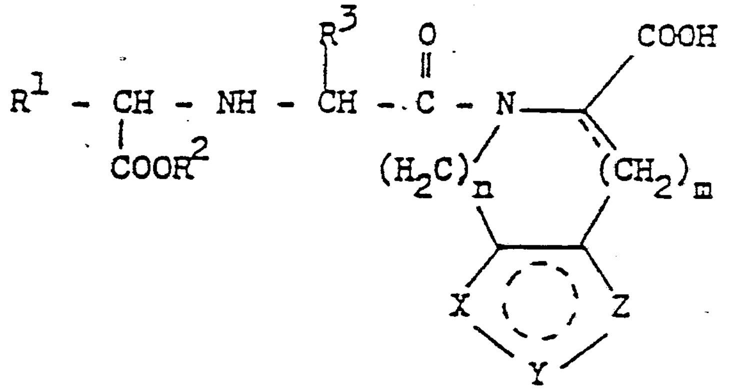

(I)

oder ihre Salze, zur Behandlung von Coronarerkrankungen oder organischem Hirnsyndrom.

## Mittel zur Behandlung von Coronarerkrankungen oder organischem Hirnsyndrom

Zahlreiche Angiotensin-Converting-Enzym-Hemmer (ACE-Hemmer) sind seit Jahren bekannt als Blutdrucksenker, deren Wirkung auf der Blockade des Renin-Angiotensin-Systems beruht. Besonders eingehend untersucht wurde das D-2-Methyl-3-mercaptopropanoyl-L-prolin, bekannt unter dem Generic Name CAPTOPRIL sowie der 1-N-[(S)-1-Ethoxycarbonyl-3-phenylpropyl]-S-alanyl-S-prolin (ENALAPRIL).

Seit einigen Jahren sind insbesondere für Captopril weitere Wirkungen aufgefunden worden. So erwies es sich als effektiv in der Behandlung der rheumatoiden Arthritis, des Raynaud-Syndroms, von Depressionen, der Herzinsuffizienz und bei Schmerzzuständen, z.B. Migräne.

Überraschenderweise wurde gefunden, daß die Verbindungen gemäß der veröffentlichten europäischen Patentanmeldung Nr. 116 842, die das Angiotensin Converting Enzym hemmen und deren Struktur sich grundlegend von der des Captoprils und des Enalaprils unterscheiden, eine sehr starke Cardioprotektion und Hirnprotektion entfalten.

Gegenstand der vorliegenden Erfindung ist daher die Verwendung einer oder mehrerer der Verbindungen, die in der veröffentlichten europäischen Patentanmeldung Nr. 116 842 beschrieben sind, für die Herstellung von pharmazeutischen Zusammensetzungen mit cardioprotektiver und/oder hirnprotektiver Wirkung, d.h. Verbindungen der Struktur

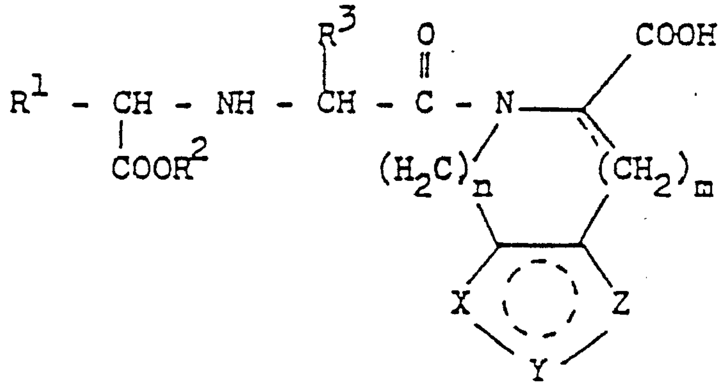

(I)

in der

$R_1$ Wasserstoff, eine Alkylgruppe mit 1 bis 6 Kohlenstoffatomen oder eine Phenylalkylgruppe mit 7 bis 12 Kohlenstoffatomen ist,

$R_2$ Wasserstoff, eine Alkylgruppe mit 1 bis 4 Kohlenstoffatomen oder eine Phenylalkylgruppe mit 7 bis 12 Kohlenstoffatomen ist,

$R_3$ Wasserstoff oder eine Alkylgruppe mit 1 bis 6 Kohlenstoffatomen ist,

n und m jeweils 0, 1 oder 2 ist, wobei die Summe aus n und m 1 oder 2 ist,

X, Y und Z unabhängig voneinander Sauerstoff, Schwefel, -N =, $NR^4$, $CR^5$, $CHR^5$, -$CH(R_5)$-$CH(R_5)$-oder -$C(R_5)$ = $C(R_5)$-bedeutet, mit der Maßgabe, daß (a) jeweils nur einer der Reste X, Y und Z Sauerstoff, Schwefel, -N =, -$CH(R_5)$-$CH(R_5)$-oder -$C(R_5)$ = $C(R_5)$-sein kann und unabhängig davon (b) nur ein oder zwei der Reste X, Y und Z $NR^4$ bedeuten können,

$R_4$ Wasserstoff oder eine Alkylgruppe mit 1 - 4 Kohlenstoffatomen ist und

$R_5$ Wasserstoff oder zusammen mit einem vicinal stehenden Rest $R_5$ einen Phenylring bedeutet,

die Linie --eine fakultative Doppelbindung ist, wenn sowohl m als auch n eins bedeutet, sowie deren Salze.

Die Verbindungen der Formel I weisen im allgemeinen mehrere Asymmetriezentren auf und liegen daher als Diastereomere oder in Form ihrer Racemate beziehungsweise ihrer racemischen Gemische vor. Die Erfindung umfaßt sowohl die Benutzung der racemischen Gemische als auch die der einzelnen Diastereomeren. Bevorzugt werden diejenigen Enantiomeren, bei denen die asymmetrischen C-Atome in der S-Konfiguration vorliegen. Die erwähnten Racemate können in üblicher Weise, z.B. durch fraktionierte Kristallisation, durch chemische oder durch biochemische Spaltung, in ihre sterisch einheitlichen Formen angereichert oder rein erhalten werden.

Die Verbindungen der Formel I können als innere Salze oder, falls freie Carboxylgruppen vorhanden sind, als Alkali-oder Erdalkalisalze z.B. als Natrium-, Kalium-, Magnesium-oder Calciumsalz und als

physiologisch unbedenkliche Salze mit Aminen wie Trimethylamin oder Dicyclohexylamin vorliegen. Ferner kann eine vorhandene freie Aminogruppe mit einer Mineralsäure, wie Salzsäure oder Bromwasserstoffsäure, oder einer organischen Säure, beispielsweise Essigsäure, zu einem Salz umgesetzt werden.

Von den Verbindungen der allgemeinen Formel I können folgende als besonders interessant hervorgehoben werden:

Verbindung der Formel I, worin
- die Summe von m und n 2 ist;

und/oder
- der an die Pyrrolidin - beziehungsweise Piperidincarbonsäure ankondensierte Ring, Furan, Pyrrol, Thiophen, Indol, Imidazol, Thiazol, Pyridin oder Isochinolin, vorzugsweise Thiophen, Imidazol oder Indol ist;

und/oder
- $R_1$ Phenylalkyl mit 7 bis 12 Kohlenstoffatomen, vorzugsweise Phenylethyl, ist;

und/oder
- $R_2$ Wasserstoff oder Alkyl mit 1 bis 4 Kohlenstoffatomen, vorzugsweis Wasserstoff oder Ethyl, ist;

und/oder
- $R_3$ Methyl ist.

Hervorzuheben sind somit Verbindungen der allgemeinen Formel I, die eine Gruppe

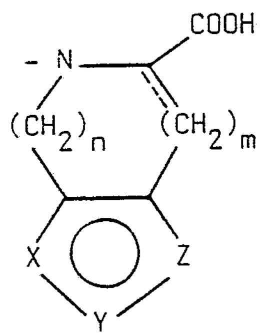

enthalten, worin
die Summe von m und n zwei ist; vorzugsweise m null oder eins ist und n eins oder zwei;
die Linie ---eine fakultative Doppelbindung bedeutet und
X, Y und Z eine der folgenden Bedeutungen haben

a) eine der Gruppen X, Y und Z ist S, die beiden anderen sind CH;

b) eine der Gruppen X, Y und Z ist NH oder $NCH_3$, die beiden anderen sind CH;

c) eine der Gruppen X und Z ist NH, die andere ist zusammen mit Y die Gruppe

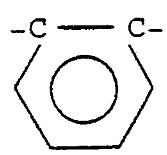
;

d) eine der Gruppen X und Z ist NH, die andere -N =, Y ist CH.

Spezifische Beispiele von Verbindungen der allgemeinen Formel I sind:

N-[N-(1-L-Ethoxycarbonyl-3-phenylpropyl)-L-alanyl]-4,5,6,7-tetrahydro-thieno[2,3-c]pyridin-7-L-carbonsäure
N-[N-(1-L-Carboxy-3-phenylpropyl)-L-alanyl]-4,5,6,7-tetrahydrothieno[2,3-c]pyridin-7-L-carbonsäure
N-[N-(1-L-Ethoxycarbonyl-3-phenylpropyl)-L-alanyl]-4,5,6,7-tetrahydro-thieno[3,2-c]pyridin-4-L-carbonsäure
N-[N-(1-L-Carboxy-3-phenylpropyl)-L-alanyl]-4,5,6,7-tetrahydrothieno[3,2-c]pyridin-4-L-carbonsäure
N-[N-(1-L-Carboxy-3-phenylpropyl)-L-alanyl]-4,5,6,7-tetrahydrothieno[3,2-c]pyridin-6-L-carbonsäure
N-[N-(1-L-Carboxy-3-phenylpropyl)-L-alanyl]-4,5,6,7-tetrahydrothieno[2,3-c]pyridin-5-L-carbonsäure
N-[N-(1-L-Carboxy-3-phenylpropyl)-L-alanyl]-4,5,6,7-tetrahydro-1H-imidazo[4,5-c]pyridin-6-L-carbonsäure
N-[N-(1-L-Carboxy-3-phenylpropyl)-L-alanyl]-2,3,4,9-tetrahydro-1H-pyrido[3,4-b]indol-1-L-carbonsäure
N-[N-(1-L-Carboxy-3-phenylpropyl)-L-alanyl]-2,3,4,9-tetrahydro-1H-pyrido[3,4-b]indol-3-L-carbonsäure
N-[N-(1-L-Carboxy-3-phenylpropyl)-L-alanyl]-4,5-dihydro-thieno [3,2-c]pyridin-6-carbonsäure
N-[N-(1-L-Carboxy-3-phenylpropyl)-L-alanyl]-6,7-dihydro-thieno [2,3-c]pyridin-5-carbonsäure

N-[N-(1-L-Ethoxycarbonyl-3-phenylpropyl)-L-alanyl]-4,5,6,7-tetrahydro-1H-imidazo[4,5-c]pyridin-6-L-carbonsäure

N-[N-(1-L-Carboxy-3-phenylpropyl)-L-alanyl]-4,5,6,7-tetrahydro-1-methylpyrrolo[3,2-c]pyridin-4-L-carbonsäure

CARDIOPROTEKTIVE WIRKUNG

Überraschenderweise wurde gefunden, daß die Verbindungen der allgemeinen Formel I und ihre Salze eine deutliche cardioprotektive Wirkung aufweisen, die wie folgt bestimmt wurde.

1. Bekanntlich ist der myocardiale $Ca^{2+}$ Gehalt ein Maß für die hypoxische bzw. die durch toxische Catecholamindosen hervorgerufene Herzschädigung (Higgins et al. Mol. Cell. Cardiol. 10. 427-438, 1984; Nakanishi et. al., Am. J. Physiol. 242, 437-449, 1982; Fleckenstein A., Vorträge der Erlanger Physiol. Tagung 1970, Edit. Keidel, Springer Verl. Berlin; Heidelberg, New York, 1971). Umgekehrt ist die Inhibition der hypoxischen oder Isoprenalin-bedingten myocardialen Calciumaufnahme ein Maß für die cardioprotektive Effektivität von Calciumantagonisten (Fleckenstein s.o), von Calmodulinhibitoren (Higgins s.o.) und anderen Pharmaka, z.B. Betaadrenolytika (Arndts, Arzneimittelforschung, 25, 1279-1284 (1975)). Die cardioprotektive Wirkung wurde an wachen Ratten nach oraler Wirkstoffgabe anhand der von Arndts (s.o.) beschriebenen Methoden festgestellt und die Wirkungsstärke der Testsubstanzen als $H_{50}$-Werte angegeben; dieser Wert entspricht der Dosis, die die durch eine Gabe von 30 mg/kg s.c. Isoprenalin bedingte myocardiale Radiocalciumaufnahme bis 50 % hemmt. Hier erwiesen sich die vorliegenden Verbindungen als wirksamer als die bekannten Handelsprodukte Captopril und Enalapril. Die Ergebnisse sind in Tabelle I dargestellt.

Tabelle 1

| Verbindung | $H_{50}$ [mg/kg] |
|---|---|
| A | 0,63 |
| D | 1,32 |
| E | 1,10 |
| Captopril | 1,52 |
| Enalapril | 1,59 |

Verbindungen A - E:

A: N-[N-(1-L-Ethoxycarbonyl-3-phenylpropyl)-L-alanyl]-4,5,6,7-tetrahydro-thieno[2,3-c]pyridin-7-L-carbonsäure

D: N-[N-(1-L-Carboxy-3-phenylpropyl)-L-alanyl]-4,5,6,7-tetrahydro-1H-imidazo[4,5-c]pyridin-6-L-carbonsäure

E: N-[N-(1-L-Carboxy-3-phenylpropyl)-L-alanyl]-4,5,6,7-tetrahydro-thieno[3,2-c]pyridin-6-L-carbonsäure

Bei in vitro oder ex vivo Versuchen an ischämischen Langendorff-Herzen bewirken die Substanzen, z.B. die oben genannte Verbindung A, nach Reperfusion eine zeitliche Verkürzung des Wiederauftretens regelmäßiger Kontraktionen. Hieraus läßt sich eine protektive Wirksamkeit der Substanzen auf Herzschädigungen während Hypoxie oder Ischämie ableiten. In diesen Testanordnungen wirken Captopril und Enalapril deutlich schwächer.

2. In einer weiteren Testanordnung wurde die Wirkung und der Mechanismus der Verbindungen auf die $Ca^{2+}$-Fluxe wie folgt nachgewiesen:
Die Bestimmung des $Ca^{2+}$-Efflux wurde nach der Methode von C. van Breemen, P. Aarsonson, R. Loutzenheiser und K. Meisheri (Chest 78, 157S-165S (1980) und von R. Casteels und G.Droogman (J.Physiol. 317, 263-276 (1981)) durchgeführt. Mit dem $Ca^{2+}$-Efflux wird die intrazelluläre $Ca^{2+}$-Freisetzung auf eine Rezeptorstimulation gemessen. Damit ist eine quantitative Beurteilung des Ausmaßes der Rezeptor-vermittelten Wirkung möglich.

Untersuchungen zum $Ca^{2+}$-Efflux an der Kaninchen-Aorta ergaben, daß die Verbindungen der Formel I den durch Kaliumionen und Noradrenalin stimulierten $Ca^{2+}$-Efflux hemmen.

Versuche mit der oben genannten Verbindung A (N-[N-(1-L-Ethoxycarbonyl-3-phenylpropyl)-L-alanyl]-4,5,6,7-tetrahydro-thieno[2,3-c]pyridin-7-L-carbonsäure ergaben: Die Hemmung des durch $10^{-5}$ mol/l Noradrenalin stimulierten $Ca^{2+}$ Efflux liegt bei 25, 24 und 30 % mit der oben angeführten Verbindung A in den entsprechenden Konzentrationen $10^{-6}$, $10^{-5}$ und $10^{-4}$ mol/l. Das weist auf eine grundsätzlich nur partielle Hemmung hin. Der $Ca^{2+}$-Influx durch $K^+$ Depolarisation wird zu 38% gehemmt ($10^{-5}$ mol/l der Verbindung A).

Eine Beeinflussung des Angiotensin I-converting Enzyms spielt in dieser Testanwendung keine Rolle.

Dies bedeutet, daß die cardioprotektive Wirkung dieser Verbindungen auch durch eine zusätzliche Sympathikushemmung interpretiert werden kann.

Die partielle Einschränkung der Noradrenalin Stimulation repräsentiert eine entscheidende Modulation der Sympathikus aktivität. Auch die Hemmung des Ca-Einstroms durch Depolarisation ist insbesondere am Herzen cytoprotektiv.

## HIRNPROTEKTIVE WIRKUNG

Daneben weisen die Substanzen eine deutliche ZNS-Wirksamkeit auf. Es ergaben sich Hinweise auf hirnprotektive Eigenschaften und auf eine günstige Beeinflussung mnestischer Funktionen, die wie folgt bestimmt werden können:

1. HYPOXIE-TOLERANZ, d.h. bei der Prüfung der Überlebensfähigkeit von Tieren in einer geschlossenen Kammer.

### Methodik

Eine aus Plexiglas hergestellte, durchsichtige, mit einem Deckel verschließbare Kammer (28 cm × 40 cm × 20 cm; Gesamtvolumen 22,4 l) ist in 2 Hälften geteilt, in die je 10 männliche oder weibliche Mäuse (Chbl: NMRI; 20-25 g Körpergewicht) gesetzt werden. Die Kammer wird mit einem Gasgemisch, bestehend aus 96,5 % $N_2$ und 3,5 % $O_2$ durchströmt; das Strömungsvolumen beträgt 12 l/min

Eine der beiden Gruppen erhält Prüfsubstanzen, die andere das Vehikel (0,5 % Tylose), die 24 h, 16 h und 30 min vor dem Test oral appliziert werden. Etwa 6 bis 7 Minuten nach Beginn der Durchströmung der Kammer sterben die ersten Tiere. Das Experiment wird beendet, wenn in der Kontrollgruppe nur noch 2-3 Tiere Zeichen von Leben zeigen. Ohne die Tiere zu berühren, wird 15 min gewartet und dann die Zahl der überlebenden Tiere in den beiden Hälften der Kammern endgültig festgestellt. Die statistische Signifikanz der Differenz der überlebenden Tiere in beiden Gruppen kann mit dem "Exakten Test von Fisher für 2 × 2 Feldertafeln" festgestellt werden. Verbindungen der Formel I erhöhen statistisch signifikant die Zahl der überlebenden Tiere.

2. Scopolamin-Test

### Methodik

Durch den cholinerg-muskarinen Antagonisten Scopolamin wird bei Mensch und Tier eine vorübergehende Störung des Überganges von Inhalten des Kurzzeitgedächtnisses in das Langzeitgedächtnis hervorgerufen. Die auch klinisch bei gesunden Probanden durch Scopolamin auslösbaren,

mnestischen Störungen sind in ihrer Ausprägung den psychopathologischen Veränderungen bei Patienten mit organischem Hirnsyndrom ähnlich (Drachman & Leavitt, 1974).

Der Scopolamin-Test wird an Gruppen von jeweils 10 männlichen, nicht nüchternen, naiven Mäusen (Chbl:NMRI; Körpergewicht 35 g) mit Hilfe einer passiven Vermeidereaktion durchgeführt. Das Testgerät besteht aus einer Skinner Box (30 cm × 26 cm × 29 cm) mit einem elektrifizierbaren Gitterboden und einer Plastikplattform (5 cm × 5 cm) an einer Seitenwand. Die Tiere werden einzeln in die Skinner Box gesetzt. Nach einer Orientierungsperiode von 30 sec werden kurze, unterbrochene Stromstöße von ca. 0,8 mA so oft an den Gitterboden der Box gelegt, bis das Tier gelernt hat, daß das Aufsuchen und Verweilen auf der Plastikplattform vor dem Stromstoß Sicherheit gewährt. Tiere, welche dies innerhalb von 120 sec nicht lernen, werden nicht weiter verwendet. Die anderen Mäuse werden unmittelbar nach Erlernen der passiven Vermeidereaktion in drei Gruppen (10 Tiere pro Gruppe) eingeteilt. Eine Gruppe erhält Scopolaminhydrobromid (0,6 mg/kg s.c.) und oral Vehikel (0,5 % Tylose); die zweite Gruppe Scopolaminhydrobromid sowie die zu testende Substanz per os in 0,5 % Tylose und die dritte Gruppe lediglich 0,5 % Tylose p.o.. Eine Stunde nach Application wird jedes Tier einzeln auf die Plattform gesetzt, um das Erinnerungsvermögen an die erlernte Vermeidereaktion zu prüfen. Das Kriterium besteht in der Bestimmung, ob die Maus während mindestens 60 sec. auf der Plattform verbleibt (Ja-Antwort) oder nicht (Nein-Antwort).

Etwa 75% der Tiere, welche ausschließlich mit 0,5 % Tylose behandelt wurden, verbleiben bei der abschließenden Prüfung länger als 60 sec. auf der Plattform: ein Ergebnis, welches als Ausdruck des guten Erinnerungsvermögens an die erlernte Erfahrung gedeutet werden kann. Nach Gabe von Scopolamin und Tylose verbleiben durchschnittlich etwa 25 % der Tiere auf der Plattform; dies zeigt die erhebliche Beeinträchtigung des Erinnerungsvermögens nach Gabe von Scopolamin. Verbindungen der Formel I antagonisieren die Scopolaminwirkung statistisch signifikant. Zum Beispiel ist die oben genannte Verbindung A (N-[N-(1-L-Ethoxycarbonyl-3-phenylpropyl)-L-alanyl]-4,5,6,7-tetrahydro-thieno[2,3-c]-pyridin-7-L-carbonsäure) bei einer Dosis über 0,1 mg/kg p.o. (insbesondere ab 0,5 mg/kg p.o.) wirksam. In Gegensatz dazu sind Captopril bei Dosen von 10 und 30 mg/kg p.o. und Enalapril bei einer Dosis von 5 mg/kg p.o. unwirksam.

Die vorliegenden Verbindungen können allein für die neuen Indikationen oder in Kombination mit anderen erfindungsgemäßen Wirkstoffen, gegebenenfalls auch in Kombination mit weiteren pharmakologisch aktiven Wirkstoffen, z.B. mit antihypertensiv wirkenden Stoffen, zur Anwendung gelangen. Die Anwendung kann parenteral oder vorzugsweise oral erfolgen. Besonders geeignete Anwendungsformen sind beispielsweise Tabletten, Kapseln, Pulver usw. Eine wirksame Dosis der Verbindungen liegt bei oraler Anwendung bei Menschen zwischen 5 und 100 mg, vorzugsweise zwischen 10 und 50 mg pro Tag.

Für die Anwendung in der Therapie werden die neuen Verbindungen mit üblichen pharmazeutischen Füll-oder Trägerstoffen, Streck-, Spreng-, Binde-, Gleit-, Dickungs-oder Verdünnungsmitteln gemischt.

Als pharmazeutische Zubereitungsformen kommen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretica, Diuretica und/oder Antihypertonica zugefügt werden können.

Die vorliegenden Verbindungen können durch die in der veröffentlichten europäischen Patentanmeldung Nr. 116 842 beschriebenen Verfahren hergestellt werden.

Pharmazeutische Anwendungsbeispiele

a) Dragees

1 Drageekern enthält:

| | |
|---|---|
| Wirkstoff | 10,0 mg |
| Milchzucker | 60,0 mg |
| Maisstärke | 35,0 mg |
| Gelatine | 3,0 mg |
| Magnesiumstearat | 2,0 mg |
| | 110,0 mg |

Herstellung:

Die Mischung der Wirksubstanz mit Milchzucker und Maisstärke wird mit einer 10%igen wässerigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragées werden mit Bienenwachs poliert.

b) Tabletten

| | |
|---|---|
| Wirkstoff | 10,0 mg |
| Milchzucker | 70,0 mg |
| Maisstärke | 50,0 mg |
| lösliche Stärke | 7,0 mg |
| Magnesiumstearat | 3,0 mg |
| | 140,0 mg |

Herstellung:

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | |
|---|---|---|
| Wirkstoff | | 5,0 mg |
| Äthanolamin | | 60,0 mg |
| Natriumchlorid | | 20,0 mg |
| destilliertes Wasser | ad | 2 ml |

Herstellung:

Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 5 mg Wirkstoff.

d) Kapseln

| | |
|---|---|
| Wirkstoff | 10,0 mg |
| Milchzucker | 250,0 mg |
| Maisstärke | 40,0 mg |
| Talk | 10,0 mg |
| | 310,0 mg |

Herstellung:

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff | 0,1 g |
| Kakaobutter (Fp. 36-37°C) | 1,6 g |
| Carnaubawachs | 0,1 g |
| | 1,8 g |

Herstellung:

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen lassen.

Als Wirkstoff sind in diesen Beispielen die oben definierten Verbindungen, vorzugsweise Verbindungen A-E, zu verwenden.

**Ansprüche**

1) Die Verwendung einer Verbindung gemäß Formel I

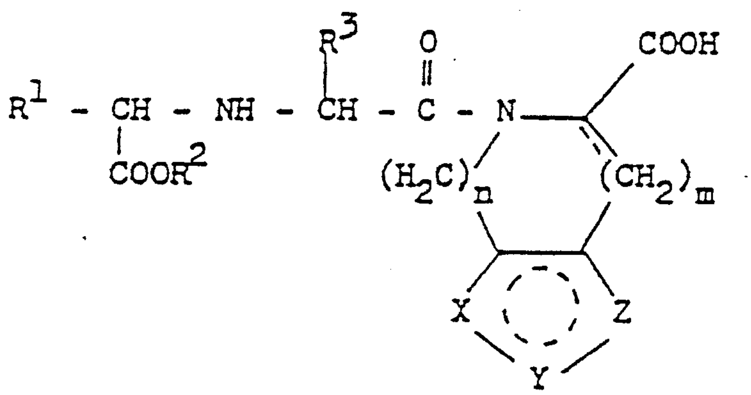

(I)

in der

$R_1$ Wasserstoff, eine Alkylgruppe mit 1 bis 6 Kohlenstoffatomen oder eine Phenylalkylgruppe mit 7 bis 12 Kohlenstoffatomen ist,

$R_2$ Wasserstoff, eine Alkylgruppe mit 1 bis 4 Kohlenstoffatomen oder eine Phenylalkylgruppe mit 7 bis 12 Kohlenstoffatomen ist,

$R_3$ Wasserstoff oder eine Alkylgruppe mit 1 bis 6 Kohlenwasserstoffatomen ist,

n und m jeweils 0, 1 oder 2 ist, wobei die Summe aus n und m 1 oder 2 ist,

X, Y und Z unabhängig von einander Sauerstoff, Schwefel, -N=, $NR^4$, $CR^5$, $CHR^5$, -CH($R_5$)-CH($R_5$)-oder -C($R_5$) = C($R_5$)-bedeuten, mit der Maßgabe, daß (a) jeweils nur einer der Reste X, Y und Z Sauerstoff, Schwefel, -N=, -CH($R_5$)-CH($R_5$)-oder -C($R_5$) = C($R_5$)-sein kann und unabhängig davon (b) nur ein oder zwei der Reste X, Y und Z $NR^4$ bedeuten können,

$R_4$ Wasserstoff oder eine Alkylgruppe mit 1 - 4 Kohlenstoffatomen ist und

$R_5$ Wasserstoff oder zusammen mit einem vicinal stehenden Rest $R_5$ einen Phenylring bedeutet,

die Linie --eine fakultative Doppelbindung ist, wenn sowohl m als auch n eins bedeutet, oder deren Salze für die Herstellung von pharmazeutischen Zusammensetzungen mit cardioprotektiver und/oder hirnprotektiver Wirkung.

2) Verwendung nach Anspruch 1, dadurch gekennzeichnet, daß in der Verbindung der Formel I $R_1$ Phenylalkyl mit 7 bis 12 Kohlenstoffatomen ist.

3) Verwendung nach Anspruch 2, dadurch gekennzeichnet, daß $R_1$ Phenylethyl ist.

4) Verwendung nach einem der Ansprüche 1 bis 3, dadurch gekennzeichnet, daß $R_2$ Wasserstoff oder Alkyl mit 1 bis 4 Kohlenstoffatomen ist.

5) Verwendung nach Anspruch 4, dadurch gekennzeichnet, daß $R_2$ Wasserstoff oder Ethyl ist.

6) Verwendung nach einem der Ansprüche 1 bis 5 dadurch gekennzeichnet, daß $R_3$ Methyl ist.

7) Verwendung nach einem der Ansprüche 1 bis 6, dadurch gekennzeichnet, daß in der Verbindung der Formel I die Summe von m und n 2 ist.

8) Verwendung nach einem der Ansprüche 1 bis 7, dadurch gekennzeichnet, daß in der Verbindung der Formel I der an die Pyrrolidin - beziehungsweise Piperidincarbonsäure ankondensierte Ring Furan, Pyrrol, Thiophen, Indol, Imidazol, Thiazol, Pyridin oder Isochinolin ist.

9) Verwendung nach Anspruch 8, dadurch gekennzeichnet, daß der ankondensierte Ring Thiophen, Imidazol oder Indol ist.

10) Verwendung nach einem der Ansprüche 1 bis 9, dadurch gekennzeichnet, daß in der Verbindung der Formel I alle asymmetrischen C-Atome in der S-Konfiguration vorliegen.

11) Verwendung nach Anspruch 1, dadurch gekennzeichnet, daß die Verbindung der Formel I
N-[N-(1-L-Ethoxycarbonyl-3-phenylpropyl)-L-alanyl]-4,5,6,7-tetrahydro-thieno[2,3-c]pyridin-7-L-carbonsäure,
N-[N-(1-L-Carboxy-3-phenylpropyl)-L-alanyl]-4,5,6,7-tetrahydro -thieno[2,3-c]pyridin-7-L-carbonsäure,
N-[N-(1-L-Ethoxycarbonyl-3-phenylpropyl)-L-alanyl]4,5,6,7-tetrahydro-thieno[3,2-c]pyridin-4-L-carbonsäure,
N-[N-(1-L-Carboxy-3-phenylpropyl)-L-alanyl]4,5,6,7-tetrahydro-thieno[3,2-c]pyridin-4-L-carbonsäure,
N-[N-(1-L-Carboxy-3-phenylpropyl)-L-alanyl]-4,5,6,7-tetrahydro-thieno[3,2-c]pyridin-6-L-carbonsäure,
N-[N-(1-L-Carboxy-3-phenylpropyl)-L-alanyl]-4,5,6,7-tetrahydro-thieno[2,3-c]pyridin-5-L-carbonsäure,
N-[N-(1-L-Carboxy-3-phenylpropyl)-L-alanyl]-4,5,6,7-tetrahydro-1H-imidazo[4,5-c]pyridin-6-L-carbonsäure,
N-[N-(1-L-Carboxy-3-phenylpropyl)-L-alanyl]2,3,4,9-tetrahydro-1H-pyrido[3,4-b]indol-1-L-carbonsäure,
N-[N-(1-L-Carboxy-3-phenylpropyl)-L-alanyl]-2,3,4,9-tetrahydro-1H-pyrido[3,4-b]indol-3-L-carbonsäure,
N-[N-(1-L-Carboxy-3-phenylpropyl)-L-alanyl]-4,5-dihydro-thieno[3,2-c]pyridin-6-carbonsäure,
N-[N-(1-L-Carboxy-3-phenylpropyl)-L-alanyl]-6,7-dihydro-thieno[2,3-c]pyridin-5-carbonsäure,
N-[N-(1-L-Ethoxycarbonyl-3-phenylpropyl)-L-alanyl]-4,5,6,7-tetrahydro-1H-imidazo[4,5-c]pyridin-6-L-carbonsäure oder
N-[N-(1-L-Carboxy-3-phenylpropyl)-L-alanyl]-4,5,6,7-tetrahydro-1-methylpyrrolo[3,2-c]pyridin-4-L-carbonsäure ist.